# EUROPEAN PATENT APPLICATION

(11) **EP 3 756 651 A1**
(43) Date of publication of application: **30.12.2020**
(21) Application number: 20179945.9
(22) Date of filing: 15.06.2020
(51) Int. Cl.: A61K 9/00, A61K 9/14, A61K 33/24, A61K 47/12, A61K 47/18, A61K 47/20, A61K 47/36, A61K 47/50, A61K 9/08

(54) **OPHTHALMIC PRODUCT WITH CORNEA REPAIR FUNCTION**

(30) Priority: 27.06.2019 TW 108122577
(71) Applicant: Pegavision Corporation, Taoyuan City 333 (TW)
(72) Inventor: CHEN, CHUN-HAN, TAOYUAN CITY 333 (TW); GAO, WAN-YING, TAOYUAN CITY 333 (TW)
(74) Representative: Durán-Corretjer, S.L.P.

(57) **Abstract**

An ophthalmic product (300, 400) having a cornea repair function includes an ophthalmic composition. The ophthalmic composition includes gold nanoparticles (100) serving as the main repairing ingredient and at least one auxiliary repairing ingredient. An effective concentration of the gold nanoparticles (100) is from 0.01 ppm to 3000 ppm. The content of the at least one auxiliary repairing ingredient is greater than 0 wt % and less than 20 wt % based on 100 wt % of the ophthalmic composition.

## Description

### CROSS-REFERENCE TO RELATED PATENT APPLICATION

This application claims the benefit of priority to Taiwan Patent Application No. 108122577, filed on June 27, 2019. The entire content of the above identified application is incorporated herein by reference. Some references, which may include patents, patent applications and various publications, may be cited and discussed in the description of this disclosure. The citation and/or discussion of such references is provided merely to clarify the description of the present disclosure and is not an admission that any such reference is "prior art" to the disclosure described herein. All references cited and discussed in this specification are incorporated herein by reference in their entireties and to the same extent as if each reference was individually incorporated by reference.

### FIELD OF THE DISCLOSURE

The present disclosure relates to an ophthalmic product, and more particularly to an ophthalmic product with a cornea repair function such as the related products of contact lenses or ophthalmic pharmaceutical products.

### BACKGROUND OF THE DISCLOSURE

The cornea, being located at the forefront of the eyeball, is transparent and has no blood vessels. The cornea receives nutrients not only from the aqueous humor but also from capillary networks in a periphery thereof. The nerve endings of the cornea are very sensitive, and once foreign objects contact the cornea, the eyelids would close involuntarily to protect the eye. In addition to the protection of in-eye structures, the cornea also plays an important role in the eye's refractive system, which is similar to the function of the lens of a camera.

The majority of people with poor vision are in the habit of wearing contact lenses for a long time. However, the eyes of a contact lens wearer may suffer from corneal inflammation and injury with an increase of wear time, caused by scratches to the cornea or contact lens-related corneal infections. A minor corneal injury will be repaired automatically within 24 hours, but a severe corneal laceration requires an appropriate therapeutic treatment with a doctor's diagnosis. In addition, office workers often stare at a computer screen for long periods of time as required by the job, which may result in dry eye. Under such a situation, the eyeball may lose a natural protective film due to a reduced wetness, which increases the probability of corneal inflammation.

Therefore, there is a need in everyday life for a novel ophthalmic product, which can not only prevent and treat common cornea injuries.

### SUMMARY OF THE DISCLOSURE

In response to the above-referenced technical inadequacies, the present disclosure provides an ophthalmic product having a cornea repair function, which can allow a user's eyes to stay healthy and comfortable.

In one aspect, the present disclosure provides an ophthalmic product having a cornea repair function, which includes an ophthalmic composition. The ophthalmic composition includes gold nanoparticles and at least one auxiliary repairing ingredient. An effective concentration of the gold nanoparticles is from 0.01 ppm to 3000 ppm. The content of the at least one auxiliary repairing ingredient is greater than 0 wt % and less than 20 wt % based on 100 wt % of the ophthalmic composition.

In certain embodiments, the average particle size of the gold nanoparticles is from 0.01 nm to 100 nm, and the effective concentration of the gold nanoparticles is from 0.05 ppm to 1600 ppm. The content of the at least one auxiliary repairing ingredient is from 0.01 wt % to 5 wt %.

In certain embodiments, the average particle size of the gold nanoparticles is from 0.5 nm to 40 nm, and the effective concentration of the gold nanoparticles is from 1 ppm to 400 ppm. The content of the at least one auxiliary repairing ingredient is from 0.05 wt % to 3 wt %.

In certain embodiments, the gold nanoparticles are each surface-modified with at least one functional molecular group that is selected from the group consisting of hydrophilic functional groups, phenol group-containing compounds, polysaccharide substances, peptide substances with at least one NH2 or COOH group and thiol ligands.

In certain embodiments, the content of the at least one functional molecular group is from 0.01 wt % to 5 wt % based on 100 wt % of the ophthalmic composition.

In certain embodiments, the hydrophilic functional groups include OH group, CONH group, CONH2 group and COOH group.

In certain embodiments, the phenol group-containing compound include monophenol, polyphenol and flavonoid compounds.

In certain embodiments, the polysaccharide substances include uronic acids, methyl carboxylic acid chitin, methyl carboxylic acid chitosan, alginic acid and hyaluronic acid.

In certain embodiments, the peptide substances have a molecular weight from 300 Daltons to 300,000 Daltons.

In certain embodiments, the thiol ligands include lipoic acid and dihydrolipoic acid.

In certain embodiments, the at least one auxiliary repairing ingredient is selected from chondroitin sulfate, α-lipoic acid, 2-aminoethanesulfonic acid and potassium L-aspartate.

In certain embodiments, the ophthalmic composition has a pH from 6 to 8 and an osmotic pressure from 240 osmol/kg to 400 osmol/kg.

In certain embodiments, the ophthalmic composition is in a solution, gel or ointment form.

In certain embodiments, the ophthalmic product further includes a transferring medium for transferring the ophthalmic composition to an eye.

In certain embodiments, the transferring medium is an ophthalmic substrate or dressing.

In certain embodiments, the ophthalmic product further includes a contact lens immersed in the ophthalmic composition that is in the solution form.

In another aspect, the present disclosure provides an ophthalmic product having a cornea repair function, which includes an ophthalmic composition. The ophthalmic composition includes gold nanoparticles. An effective concentration of the gold nanoparticles is from 0.01 ppm to 3000 ppm, and the average particle size of the gold nanoparticles is from 0.01 nm to 100 nm.

In certain embodiments, the average particle size of the gold nanoparticles is from 0.5 nm to 40 nm.

In certain embodiments, the effective concentration of the gold nanoparticles is from 0.05 ppm to 1600 ppm.

In certain embodiments, the effective concentration of the gold nanoparticles is from 1 ppm to 400 ppm.

One of the advantages of the present disclosure is that, the ophthalmic composition can prevent and treat common cornea injuries and relieve eye discomfort symptoms such as eye pain, photophobia, watery eyes, blurred vision, and vascular proliferation, by the features of "the ophthalmic composition includes gold nanoparticles and at least one auxiliary repairing ingredient" and "the effective concentration of the gold nanoparticles is from 0.01 ppm to 3000 ppm".

These and other aspects of the present disclosure will become apparent from the following description of the embodiment taken in conjunction with the following drawings and their captions, although variations and modifications therein may be affected without departing from the spirit and scope of the novel concepts of the disclosure.

### BRIEF DESCRIPTION OF THE DRAWINGS

The present disclosure will become more fully understood from the following detailed description and accompanying drawings.
FIG. 1 is a partial schematic view of an ophthalmic product of the present disclosure.
FIG. 2 is another partial schematic view of an ophthalmic product of the present disclosure.
FIG. 3 is still another partial schematic view of an ophthalmic product of the present disclosure.
FIG. 4 is a perspective view of an ophthalmic product according to an exemplary embodiment of the present disclosure.
FIG. 5 is a sectional view of the ophthalmic product according to the exemplary embodiment of the present disclosure.
FIG. 6 shows an ophthalmic product in a state of use according to another exemplary embodiment of the present disclosure.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

Unless defined otherwise, all technical and scientific terms used herein have the same meanings as commonly understood by one of ordinary skill in the art to which this invention belongs. As used herein, the singular forms "a," "an," and "the," are intended to include the plural forms.

Unless indicated otherwise, all percentages disclosed herein are in weight percent. The upper and lower limits of these smaller ranges may independently be included in the smaller ranges and are also encompassed within the disclosure, subject to any specifically excluded limit in the stated range.

In order to increase the protection and repair abilities of cells (e.g., cornea cells) in different layered tissues of an eye, the present disclosure provides an ophthalmic product having a cornea repair function. The ophthalmic product of the present disclosure includes an ophthalmic composition that mainly includes gold nanoparticles and at least one auxiliary repairing ingredient. When the ophthalmic product is in use, an effective amount of the gold nanoparticles and the at least one auxiliary repairing ingredient can be transferred to an eye surface area by directly or indirectly contacting the ophthalmic composition with the eye surface area. Furthermore, the gold nanoparticles and the at least one auxiliary repairing ingredient can produce the synergistic effect of cornea repair. As used herein, the term "eye surface area" includes a cornea, a conjunctiva, a tear film and their adjacent or related structures.

More specifically, the ophthalmic product of the present disclosure can be the related products of contact lenses or ophthalmic pharmaceutical products. The ophthalmic composition can be in a solution, gel or ointment form and, for example, it can serve as a package solution, a storage solution, a cleaning solution or a care solution of the contact lenses, or can serve as multifunctional eyedrops or an ophthalmic pharmaceutical preparation. However, such examples are not intended to limit the present disclosure.

In the present embodiment, the effective concentration of the gold nanoparticles can be from 0.01 ppm to 3000 ppm, preferably from 0.05 ppm to 1600 ppm, and more preferably from 1 ppm to 400 ppm. For example, the effective concentration of the gold nanoparticles is 5 ppm, 10 ppm, 15 ppm, 20 ppm, 25 ppm, 50 ppm, 75 ppm, 100 ppm, 150 ppm, 200 ppm, 250 ppm, 300 ppm or 350 ppm. As used herein, the term "effective concentration" is a concentration that can deliver a sufficient amount of the gold nanoparticles to the eye surface area to produce beneficial effects.

It has been found that the gold nanoparticles at least have the functions or effects of antioxidation, antiinflammation, antiallergy, relief, corneal repair and vascular proliferation inhibition. Therefore, the ophthalmic product, in which the ophthalmic composition includes the gold nanoparticles, can effectively maintain a user's eyes in a healthy and comfortable state.

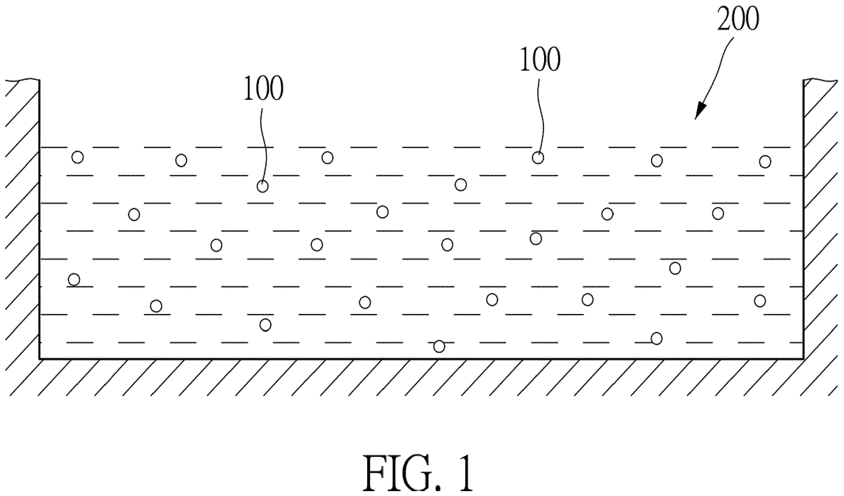

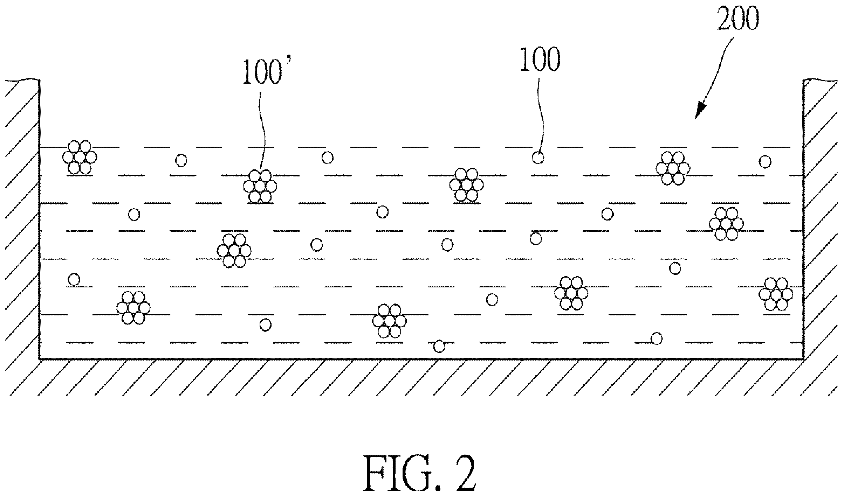

Referring to FIG. 1 and FIG. 2, the ophthalmic composition includes a dispersion medium 200 for dispersing the gold nanoparticles. The dispersion medium 200 of the ophthalmic composition can be water, but it is not limited thereto. The content of the dispersion medium 200 can be from 75 wt % to 99 wt %, preferably from 85 wt % to 99 wt %, based on 100 wt % of the ophthalmic composition. As shown in FIG. 2, a number of the gold nanoparticles 100 can be grouped together to form a gold nanocluster 100' according to practical implementations. The average particle size of the gold nanoparticles 100 or gold nanoclusters 100' is from 0.01 nm to 100 nm, and preferably from 0.5 nm to 40 nm.

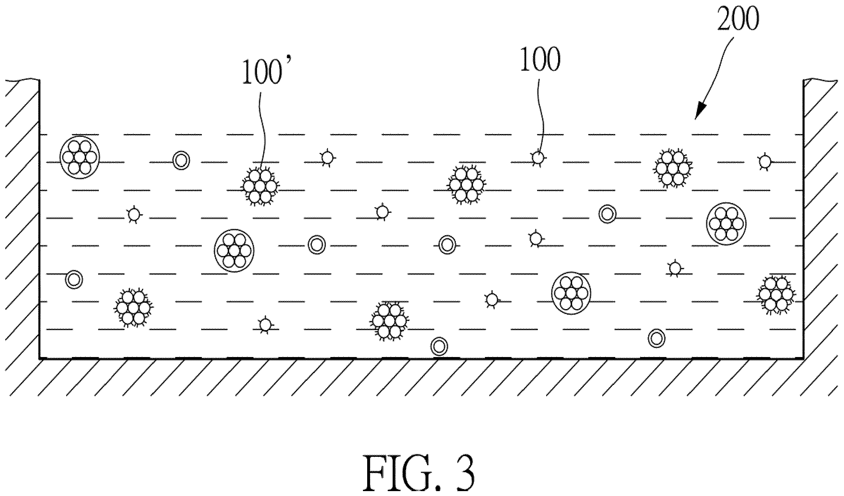

Referring to FIG. 3, the gold nanoparticles 100 or gold nanoclusters 100' can be surface-modified with at least one functional molecular group according to practical implementations. That is, the gold nanoparticles 100 or gold nanoclusters 100' have the at least one functional molecular group attached onto their surfaces to increase the functionality thereof. The at least one functional molecular group can be selected from the group consisting of hydrophilic functional groups, phenol group-containing compounds, polysaccharide substances, peptide substances with at least one NH2 or COOH group and thiol ligands, but it is not limited thereto. The content of the at least one functional molecular group can be greater than 0 wt % and less than 20 wt % based on 100 wt % of the ophthalmic composition, preferably from 0.01 wt % to 5 wt %, and more preferably from 0.05 wt % to 3 wt %.

It is worth mentioning that the gold nanoparticles 100 or gold nanoclusters 100' surface-modified with one or more hydrophilic functional groups have good hydrophilicity. The gold nanoparticles 100 or gold nanoclusters 100' surface-modified with one or more phenol group-containing compounds, preferably with monophenol, polyphenol and flavonoid compounds, can regulate the concentration of glutathione in cells. The gold nanoparticles 100 or gold nanoclusters 100' surface-modified with one or more polysaccharide substances or peptide substances with at least one NH2 or COOH group can not only meet the requirements of biological safety, but also increase the abilities of free radical resistance and moisture retention. The gold nanoparticles 100 or gold nanoclusters 100' surface-modified with one or more thiol ligands have an increased antioxidative ability.

In the present embodiment, the hydrophilic functional groups can include OH group, CONH group, CONH2 group and COOH group. The polysaccharide substances can include uronic acids, methyl carboxylic acid chitin, methyl carboxylic acid chitosan, alginic acid and hyaluronic acid. The peptide substances have a molecular weight from 300 Daltons to 300,000 Daltons. The thiol ligands can be molecules having SH groups such as lipoic acid and dihydrolipoic acid. However, such examples are not intended to limit the present disclosure.

The content of at least one the auxiliary repairing ingredient can be greater than 0 wt % and less than 20 wt %, preferably from 0.001 wt % to 5 wt %, more preferably from 0.005 wt % to 3 wt, based on 100 wt % of the ophthalmic composition. For example, the content of the auxiliary repairing ingredient is 0.01 wt %, 0.05 wt %, 0.1 wt %, 0.2 wt %, 0.3 wt %, 0.4 wt %, 0.5 wt %, 0.6 wt %, 0.7 wt %, 0.8 wt %, 0.9 wt %, 1.0 wt%, 1.5 wt %, 2.0 wt % or 2.5wt %.

In the present embodiment, the at least one auxiliary repairing ingredient can be selected from chondroitin sulfate, α-lipoic acid, 2-aminoethanesulfonic acid and potassium L-aspartate, but it is not limited thereto. It is worth mentioning that such substances can promote the microcirculation of layered tissues of an eyeball, which can promote cell metabolism, increase oxygen content in cells and nourish an eye portion, and especially, can activate cellular respiration at edges of an eye and provide cool and comfortable sensations to the eye. Furthermore, such substances and the gold nanoparticles or nanoclusters (hereinafter referred to as "a nano-gold ingredient") can work with each other under different mechanisms to produce unexpected cornea repair effects.

The ophthalmic composition can further include a buffering agent, a surfactant, a hydrophilic polymer, an active pharmaceutical ingredient and other functional additives. The buffering agent can be added to adjust the pH and osmolality of the ophthalmic composition to allow the ophthalmic composition to have desired effects, i.e., to be beneficial effects for the eyes. The pH of the ophthalmic composition can be from 6 to 8, and preferably from 7 to 8. The osmolality of the ophthalmic composition can be from 240 osmol/kg to 400 osmol/kg, and preferably from 260 osmol/kg to 340 osmol/kg.

In the present embodiment, the buffering agent can be a borate buffer or a phosphate buffer. The content of the buffering agent can be greater than 0 wt % and less than 5 wt % based on 100 wt % of the ophthalmic composition, e.g., 0.5 wt %, 1 wt %, 1.5 wt %, 2 wt %, 2.5 wt %, 3 wt %, 3.5 wt % or 4 wt %. The borate buffer may include boric acid, sodium chloride and a borate such as sodium tetraborate, but it is not limited thereto. The phosphate buffer may include sodium chloride and phosphates such as sodium dihydrogen phosphate, disodium hydrogen phosphate, potassium dihydrogen phosphate and dipotassium hydrogen phosphate, but it is not limited thereto.

The surfactant can act to remove eye discharge and improve blurred vision. Furthermore, the surfactant can act to enhance the performance of the nano-gold ingredient. The surfactant can be at least one selected from polysorbate 80 (also known as Tween 80), an alkyl sulfosuccinate (e.g., SBFA 30), sodium lauroyl lactylate, polyoxypropylene glycol, polyoxyethylene hardened castor oil and polyvinylpyrrolidone (PVP), but it is not limited thereto. The content of the surfactant can be from 0.01 wt % to 5 wt %, preferably from 0.01 wt % to 3 wt %, based on 100 wt % of the ophthalmic composition, e.g., 0.5 wt %, 1 wt %, 1.5 wt %, 2 wt % or 2.5 wt %.

The hydrophilic polymer can act to increase eye moisture. Furthermore, the hydrophilic polymer can act to enhance the slow release effect of the nano-gold ingredient and prolong the in-eye residence time of the nano-gold ingredient, so as to provide beneficial effects to the eyes. The hydrophilic polymer can be at least one selected from polyethylene glycol (PEG400), 2-methacryloyloxyethyl phosphorylcholine (MPC) and hyaluronic acid, but it is not limited thereto. The content of the hydrophilic polymer can be from 0.01 wt % to 5 wt %, preferably from 0.01 wt % to 3 wt %, based on 100 wt % of the ophthalmic composition, e.g., 0.5 wt %, 1 wt %, 1.5 wt %, 2 wt % or 2.5 wt %.

The active pharmaceutical ingredient can be added to provide antiinflammatory, antiallergic and alleviative effects. The active pharmaceutical ingredient can be at least one selected from pranoprofen, ε-aminocaproic acid, allanton, berberine, sodium azulene sulfonate, glycyrrhizic acid, sodium cromoglycate and zinc sulfate. The content of the active pharmaceutical ingredient can be from 0.001 wt % to 20 wt % based on 100 wt % of the ophthalmic composition, e.g., 0.01 wt %, 0.05 wt %, 1 wt % or 10 wt %. In the present embodiment, the ophthalmic composition can include 0.001 wt % to 5 wt % of pranoprofen, 0.001 wt % to 5 wt % of ε-aminocaproic acid, 0.001 wt % to 5 wt % of allanton, 0.001 wt % to 10 wt % of berberine, 0.001 wt % to 10 wt % of glycyrrhizic acid, 0.001 wt % to 10 wt % of sodium cromoglycate or 0.001 wt % to 10 wt % of zinc sulfate, which serve as the active pharmaceutical ingredient, but the present disclosure is not limited thereto.

The functional additives can include an antibacterial agent and a vitamin, but they are not limited thereto. The content of the functional additive can be from 0.01 wt % to 5 wt % based on 100 wt % of the ophthalmic composition. Specific examples of the antimicrobial agent include polyhexamethylene biguanide (PHMB) and its water soluble salts and polyaminopropyl biguanide (PAPB) and its water soluble salts. Specific examples of the vitamin include vitamin B6 (pyridoxine hydrochloride), vitamin B12 (cyanocobalamin) and vitamin E (synthetic dl-alpha-tocopherol). However, such examples are not intended to limit the present disclosure.

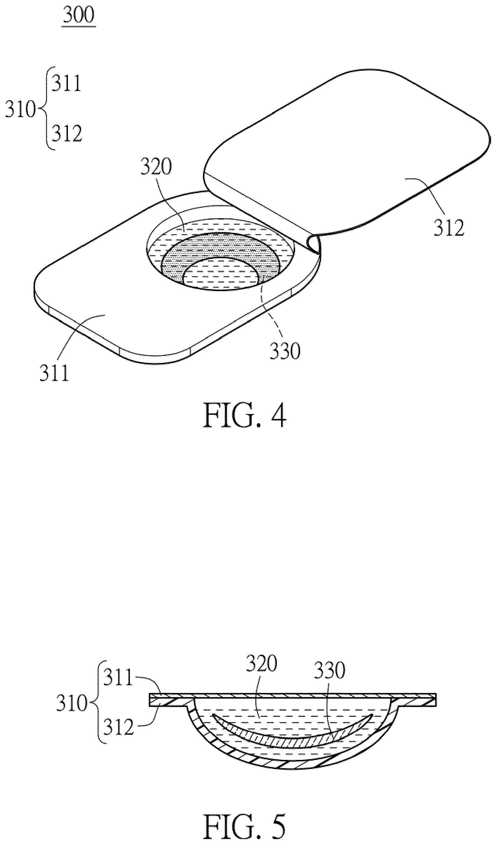

Referring to FIG. 4 and FIG. 5, an ophthalmic product 300 according to a preferable embodiment of the present disclosure is shown, which is a contact lens product including a package structure 310, a package solution 320 resulted from the ophthalmic composition and a contact lens 330. The package solution 320 and the contact lens 330 are sealed together in the package structure 310 and are sterilized (e.g., sterilized at high temperature or high pressure), in which the contact lens 330 is immersed in the package solution 320.

More specifically, the package structure 310 includes a container 311 and a cover sheet 312. The container 311 is used to accommodate the package solution 320 and the contact lens 330. The cover sheet 312 is peelably bonded to the container 311 to seal its opening. In the present embodiment, the container 311 may be made of a plastic, and provides a reasonable degree of protection to the contact lens 330. The cover sheet 312 may be made of a metal or a plastic. The contact lens 330 may be made of a hydrogel or a silicone hydrogel, and may contain one or more functional materials if necessary, such as a blue light absorbing ingredient and a UV absorbing ingredient. However, such examples are not intended to limit the present disclosure.

It is worth mentioning that when the contact lens 330 is immersed in the package solution 320, beneficial ingredients in the package solution 320 would enter the contact lens 330 or adhere onto the contact lens 330. Therefore, when the contact lens 330 is put on an eye of a person, the beneficial ingredients can be transferred to an eye surface area from the contact lens 330, so as to prevent and treat common cornea injuries and relieve eye discomfort symptoms such as eye pain, photophobia, watery eyes, blurred vision, and vascular proliferation

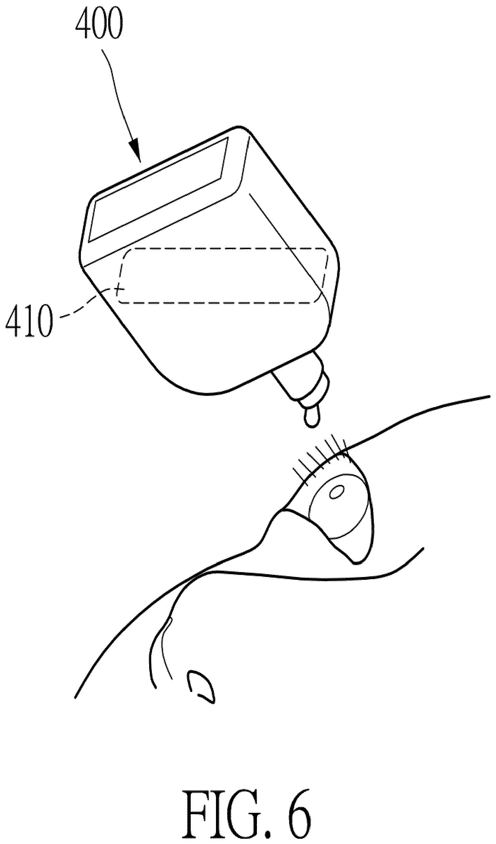

Referring to FIG. 6, an ophthalmic product 400 according to another preferable embodiment of the present disclosure is shown, which includes an ophthalmic preparation 410 resulted from the ophthalmic composition. In use, the ophthalmic preparation 410 can be transferred to an eye surface area in the form of drops, but it is not limited thereto. In other embodiments, the ophthalmic preparation 410 can be transferred to the eye surface area by a transferring medium such as an ophthalmic substrate or dressing.

### [Evaluation areas]

### Preparation of ophthalmic products:

Contact lens package solutions were prepared according to the ophthalmic compositions of Examples 1-4 and Comparative Example 1 as shown in Table 1. Hydrogel contact lenses produced by the Pegavision Corporation were respectively immersed in the contact lens package solutions. After sealing and high temperature sterilizing (125oC, 30 minutes) treatments, the ophthalmic products (i.e., contact lens products) were obtained.

The comparison between Examples 1-4 and Comparative Example 1 of Table 1 were obtained by ten clinical trial subjects each wearing the contact lenses to conduct a self-awareness evaluation by a questionnaire. Evaluation items were divided into positive and negative groups, and each thereof was scored immediately after putting on the contact lenses and after wearing for four hours. The results are shown in Table 2, in which the score for each evaluation item is an average value of ten scores.

Since the contact lenses are medical devices that must have biocompatibility, cytotoxicity is an initial test indicator. Therefore, an in-vitro cytotoxicity test in accordance with the ISO 10993-5:2009 standard is conducted to confirm whether or not test objects have cytotoxicity to mouse fibroblasts (cell line L929). The test objects include the package solutions and the contact lenses. The cytotoxicity was graded with a score of 0-4 in accordance with Table 1: "Qualitative morphological grading of cytotoxicity of extracts" of the ISO 10993-5:2009 standard; Score "0" represents no reactivity, Grade "1" represents slight reactivity and a cell variability of less than 20 %, Grade "2" represents mild reactivity and a cell variability of less than 50 %, Grade "3" represents moderate reactivity and a cell variability of less than 70 %, and Grade "4" represents severe reactivity and a nearly complete or complete destruction of cell layers. The results are shown in Table 3.

In recent years, smartphones and LED light sources which emit blue light have become more and more popular. In addition, the eyes of an outdoor worker may suffer from blue light damage as a result of long periods of direct exposure to sunlight. However, prolonged exposure to blue light may result in the damage or death of cornea cells. More severely, macular degeneration, blurred vision, distortion vision or dark shadows in central vision may occur in the eyes. Therefore, it is very important for eye health to block blue light, so that products with blue light protection have become more and more popular. The International Journal of Ophthalmology published in 2017 mentioned that eye cells contain reduced glutathione (GSH), which is an antioxidant of human body and is present in the lens, cornea, optic nerve, retina and ciliary body in high concentrations. GSH can combine with free radicals by thiol groups to form an acidic substance that is easily metabolized, thereby accelerating the excretion of the free radicals. Furthermore, thiol groups of unstable lens proteins can be inhibited, and thus the incidence rate of cataract can be reduced and the development of keratopathy and retinopathy can be controlled. These are beneficial for maintaining the transparency of the cornea or lens and tissue regeneration and repair. The ophthalmic product of the present disclosure can be used to increase the antioxidative ability of the eye surface area, maintain the concentration of reduced glutathione (GSH) in the eye cells, and block blue light, thereby effectively preventing eye diseases and protecting the eyes from blue light.

In the comparison between Example 4 and Comparative Example 1 as shown in Table 1, blue lights were used to irradiate corneal cells in the contact lens lenses, so as to quantify the content of GSH in the corneal cells in a defense mode. The degree of cell damage was observed for verification. The selected cell line was bovine cornea endothelial cells. The experimental method was to inoculate corneal endothelial cells on a 12-well cell culture plate for 12 hours. Subsequently, the corneal endothelial cells were respectively added into the contact lenses to be immersed the ophthalmic compositions of Example 4 and Comparative Example 1 and then irradiated with blue lights (3W) for 24 hours. After that, the four observed states of the cells were used to detect the GSH content of the cells, in which the damaged cells would have a reduced GSH content. The test results are shown in Table 4.

**Table 1**

| Ophthalmic composition | Examples | | | | Comparative Example |
|---|---|---|---|---|---|
| | 1 | 2 | 3 | 4 | 1 |
| Borate buffer solution | bal. | bal. | bal. | bal. | bal. |
| Hyaluronic acid | 0.01 % | 0.01 % | 0.01 % | 0.01 % | 0.01 % |
| Lipoic acid | 0.01 % | | 0.01 % | | |
| Gold nanoparticles | | 0-150 ppm | 0-150 ppm | | |
| Thiol ligand-modified old nanoparticles | | | | 0-150 ppm | |

**Table 2**

| Clinical self-awareness evaluation (Average value of ten scores) | | Evaluation time | Examples | | | | Comparative Example |
|---|---|---|---|---|---|---|---|
| | | | 1 | 2 | 3 | 4 | 1 |
| Positive | Comfort degree | Immediately after putting on contact lenses | 10 | 10 | 10 | 10 | 9 |
| | | After wearing for 4 hours | 8 | 8 | 9 | 10 | 7 |
| | Visual performance | Immediately after putting on contact lenses | 10 | 10 | 10 | 10 | 10 |
| | | After wearing for 4 hours | 7 | 9 | 9 | 9 | 7 |
| | Moisture sensation | Immediately after putting on contact lenses | 10 | 10 | 10 | 10 | 10 |
| | | After wearing for 4 hours | 7 | 8 | 8 | 8 | 6 |
| Negative | Dryness sensation | Immediately after putting on contact lenses | 0 | 0 | 0 | 0 | 0 |
| | | After wearing for 4 hours | 3 | 2 | 2 | 2 | 3 |
| | Sour sensation | Immediately after putting on contact lenses | 0 | 0 | 0 | 0 | 0 |
| | | After wearing for 4 hours | 2 | 1 | 0 | 0 | 2 |
| | Itch Sensation | Immediately after putting on contact lenses | 0 | 0 | 0 | 0 | 0 |
| | | After wearing for 4 hours | 0 | 0 | 0 | 0 | 0 |
| | Foreign matter sensation | Immediately after putting on contact lenses | 0 | 0 | 0 | 0 | 0 |
| | | After wearing for 4 hours | 3 | 2 | 1 | 1 | 4 |
| | Irritation sensation | Immediately after putting on contact lenses | 0 | 0 | 0 | 0 | 0 |
| | | After wearing for 4 hours | 0 | 0 | 0 | 0 | 0 |
| | Blurred vision | Immediately after putting on contact lenses | 0 | 0 | 0 | 0 | 0 |
| | | After wearing for 4 hours | 3 | 2 | 1 | 1 | 3 |

**Table 3**

| Cytotoxicity Grade | Examples | | | | Comparative Example |
|---|---|---|---|---|---|
| | 1 | 2 | 3 | 4 | 1 |
| Contact lens | 0 | 0 | 0 | 0 | 0 |
| Package solution | 0 | 0 | 0 | 0 | 0 |

**Table 4**

| Reduced GSH conc. (%) in cells | Examples | | | | Comparative Example |
|---|---|---|---|---|---|
| | 1 | 2 | 3 | 4 | 1 |
| Defense mode | NA | NA | NA | 97% | 20% |

The compositions of Examples 1-4 of the present disclosure, in which the gold nanoparticles have no cytotoxicity in the cytotoxicity trial, have good biological safety when used in ophthalmic products. Furthermore, the performance of the gold nanoparticles can be enhanced in the presence of the at least one auxiliary repairing ingredient, so as to eliminate or relieve negative evaluations (e.g., eye discomfort and foreign matter sensation) of long-time contact lens wearers and to maintain their eyes in a moist and comfortable state for a long period of time. It is observed from the repair trial of the corneal endothelial cells that, the repair effects on common cornea injuries resulted from Examples 1-4 are better than the repair effect on that resulted from Comparative Example 1.

One of the advantages of the present disclosure is that, the ophthalmic composition with a cornea repair function can prevent and treat common cornea injuries and relieve eye discomfort symptoms such as eye pain, photophobia, watery eyes, blurred vision, and vascular proliferation, by the features of "the ophthalmic composition includes gold nanoparticles and at least one auxiliary repairing ingredient" and "the effective concentration of the gold nanoparticles is from 0.01 ppm to 3000 ppm".

Furthermore, the gold nanoparticles at least have the functions or effects of antioxidation, antiinflammation, antiallergy, alleviation, corneal repair and vascular proliferation inhibition. Therefore, the ophthalmic product can effectively allow a user's eyes to stay healthy and comfortable. The nano-gold ingredient and the at least one auxiliary repairing ingredient (i.e., chondroitin sulfate, α-lipoic acid, 2-aminoethanesulfonic acid and/or potassium L-aspartate) can work with each other under different mechanisms to produce unexpected cornea repair effects.

In addition, the gold nanoparticles can be surface-modified with at least one functional molecular group according, i.e., the gold nanoparticles have the at least one functional molecular group attached onto their surfaces, to increase the functionality thereof.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its spirit and scope.

## Claims

1. An ophthalmic product (300, 400) having a cornea repair function **characterized by** comprising an ophthalmic composition that includes gold nanoparticles (100) and at least one auxiliary repairing ingredient, wherein an effective concentration of the gold nanoparticles (100) is from 0.01 ppm to 3000 ppm, and the content of the at least one auxiliary repairing ingredient is greater than 0 wt % and less than 20 wt % based on 100 wt % of the ophthalmic composition.

2. The ophthalmic product (300, 400) according to claim 1, wherein the average particle size of the gold nanoparticles (100) is from 0.5 nm to 40 nm, the effective concentration of the gold nanoparticles (100) is from 1 ppm to 400 ppm, and the content of the at least one auxiliary repairing ingredient is from 0.05 wt % to 3 wt %.

3. The ophthalmic product (300, 400) according to claim 1, wherein the gold nanoparticles (100) are each surface-modified with at least one functional molecular group that is selected from hydrophilic functional groups, phenol group-containing compounds, polysaccharide substances, peptide substances with at least one NH2 or COOH group and thiol ligands.

4. The ophthalmic product (300, 400) according to claim 3, wherein the content of the at least one functional molecular group is from 0.01 wt % to 5 wt % based on 100 wt % of the ophthalmic composition.

5. The ophthalmic product (300, 400) according to claim 3, wherein the hydrophilic functional groups include OH group, CONH group, CONH2 group and COOH group.

6. The ophthalmic product (300, 400) according to claim 3, wherein the phenol group-containing compounds include monophenol, polyphenol and flavonoid compounds.

7. The ophthalmic product (300, 400) according to claim 3, wherein the polysaccharide substances include uronic acids, methyl carboxylic acid chitin, methyl carboxylic acid chitosan, alginic acid and hyaluronic acid.

8. The ophthalmic product (300, 400) according to claim 3, wherein the peptide substances have a molecular weight from 300 Daltons to 300,000 Daltons.

9. The ophthalmic product (300, 400) according to claim 3, wherein the thiol ligands include lipoic acid and dihydrolipoic acid.

10. The ophthalmic product (300, 400) according to claim 1, wherein the at least one auxiliary repairing ingredient is selected from chondroitin sulfate, α-lipoic acid, 2-aminoethanesulfonic acid and potassium L-aspartate.

11. An ophthalmic product (300, 400) having a cornea repair function **characterized by** comprising an ophthalmic composition that includes gold nanoparticles (100), wherein an effective concentration of the gold nanoparticles (100) is from 0.01 ppm to 3000 ppm, and the average particle size of the gold nanoparticles (100) is from 0.01 nm to 100 nm.

12. The ophthalmic product (300, 400) according to claim 11, wherein the average particle size of the gold nanoparticles (100) is from 0.5 nm to 40 nm.

13. The ophthalmic product (300, 400) according to claim 11, wherein the effective concentration of the gold nanoparticles (100) is from 1 ppm to 400 ppm.
